# EUROPÄISCHE PATENTSCHRIFT

(11) **EP 4 140 514 B1**
(45) Veröffentlichungstag und Bekanntmachung des Hinweises auf die Patenterteilung: **10.07.2024**
(21) Anmeldenummer: 22190905.4
(22) Anmeldetag: 18.08.2022
(51) Int. Cl.: A61M 1/00, B29C 49/06

(54) **WUNDDRAINAGEBEHÄLTER**
WOUND DRAINAGE CONTAINER
RÉCIPIENT DE DRAINAGE POUR PLAIES

(30) Priorität: 20.08.2021 DE 102021121638
(43) Veröffentlichungstag der Anmeldung: 01.03.2023
(73) Patentinhaber: B. Braun Melsungen AG, 34212 Melsungen (DE)
(72) Erfinder: NAGY, Karoly, 5100 Jászberény (HU)
(74) Vertreter: Winter, Brandl - Partnerschaft mbB

(56) Entgegenhaltungen:
- EP-A1- 3 268 062
- DE-A1- 19 953 910
- GB-A- 1 550 653
- US-A- 4 828 546
- US-A- 6 047 851
- US-A1- 2012 029 486
- US-A1- 2017 157 305

## Beschreibung

### Technisches Gebiet

Die vorliegende Offenbarung betrifft Wunddrainagebehälter.

### Hintergrund der Erfindung

Bei der Versorgung von Patienten, insbesondere nach größeren operativen Eingriffen, ist es häufig notwendig, Blut und Wundsekret nach außen abzuleiten. Hierfür setzt ein Chirurg vor einer Hautnaht in einem Unterhautfettgewebe des Patienten eine Wunddrainage in die Wunde ein und Blut und Wundsekret wird wenige Zentimeter von der Wunde entfernt aus der Haut heraus durch einen (Wunddrainage-) Schlauch herausgeleitet. Der Schlauch ist mit einem Wunddrainagebehälter verbunden, der das Blut und Wundsekret auffängt.

Postoperative Wunddrainagen verbleiben für gewöhnlich für zwei bis drei Tage in dem Patienten. Es ist also davon auszugehen, dass ein mit einer Wunddrainage versorgter Patient einen gewissen Grad an aktiver und passiver Mobilität aufweist. In anderen Worten ist davon auszugehen, dass der Patient sich in diesem Zeitraum selbstständig bewegt oder von beispielsweise medizinischem Personal bewegt und/oder transportiert wird.

Bei Blut und Wundsekret handelt es sich um potenziell infektiöses Material der Schutzstufe 2 nach TRBA 250. Es ist daher von höchster Bedeutung, den Patienten, Behandler und die Umgebung vor einer Kontamination mit den in dem Wunddrainagebehälter enthaltenen Stoffen zu schützen, um eine mögliche Infektion zu verhindern. Wenn nun der Patient mit dem Wunddrainagebehälter transportiert wird oder sich fortbewegt, besteht immer die Gefahr, dass der Wunddrainagebehälter, welcher häufig an einer Außenseite eines Bettes eines Rollstuhls oder dergleichen befestigt wird, beschädigt wird und das enthaltene potenziell infektiöse Material ausläuft/austritt und damit eine Gefahr für den Patienten, die Behandler und die Umgebung bzw. Personen in der Umgebung darstellt.

GB 1 550 653 A offenbart einen Wunddrainagebehälter mit einem Deckel und einem Behälterkörper aus Kunststoff.

Es wurden diverse Versuche unternommen, um die Sicherheit des Wunddrainagebehälters, insbesondere gegen Platzen und Auslaufen, zu erhöhen. Hierfür wurde der Wunddrainagebehälter in eine Art Käfig eingehaust, was jedoch die Kosten erheblich steigerte und den Wunddrainagebehälter umständlich in der Handhabung machte, insbesondere für den Patienten selbst. Auch wurde versucht, die Wandstärke des Wunddrainagebehälters zu erhöhen, was jedoch das Zusammendrücken des Wunddrainagebehälters zur Erzeugung eines nötigen Unterdrucks in dem Wunddrainagebehälter erheblich erschwerte und die Rückstellkräfte derart erhöhte, dass der entstehende Sog zu stark war.

Ein weiterer Ansatz war, das Material des Wunddrainagebehälters mit festigkeitserhöhenden Füllstoffen zu versehen. In anderen Worten wurde ein faserverstärkter Kunststoff eingesetzt. Hier hatte sich jedoch gezeigt, dass die Füllstoffe die Transparenz des Materials des Wunddrainagebehälters derart verschlechtern, dass eine visuelle Inspektion der Flüssigkeit erheblich erschwert wurde.

### Kurzbeschreibung der Erfindung

Aufgabe der vorliegenden Erfindung ist es daher, einen Wunddrainagebehälter zur Verfügung zu stellen, der einerseits möglichst stabil gegenüber auf den Wunddrainagebehälter wirkenden Kräften ist und damit eine möglichst große Sicherheit gegenüber Auslaufen und Platzen bietet, aber nicht die Nachteile aus dem Stand der Technik mit sich bringt.

Die Aufgabe wird durch einen Wunddrainagebehälter nach Anspruch 1 gelöst. Vorteilhafte Ausgestaltungen der Erfindung sind Gegenstand der beigefügten Unteransprüche.

Insbesondere wird die Aufgabe erfindungsgemäß durch den Wunddrainagebehälter, der einen Behälterkörper und einen Verschlussdeckel aufweist, wobei der Behälterkörper mittels Kunststoff-Spritzblasformen hergestellt ist, gelöst. Der Wunddrainagebehälter ist vorgesehen und ausgebildet, Wundflüssigkeit aufzunehmen. Der Wunddrainagebehälter weist weiterhin einen Verschlussdeckel auf, der mittels Spritzgießen hergestellt ist und einen ersten Anschluss und einen zweiten Anschluss aufweist.

In anderen Worten wird ein durch Spritzblasformen (auch als "injection blowing technology" bezeichnet) gefertigter Behälter als Wunddrainagebehälter verwendet. Beim Spritzblasformen wird ein meist spritzgegossener Vorformling auf einem Spritzdorn in einer Blasform mittels Druckluft in eine Form/Geometrie geblasen. Bisher wurden als Wunddrainagebehälter hauptsächlich mittels Extrusionsblasformen hergestellte Behälter verwendet.

Da sich beim Spritzblasformen dünnere Wandstärken ergeben als beim Extrusionsblasformen scheint dieses Verfahren zunächst zur Erfüllung der Aufgabe, nämlich die Sicherheit des Wunddrainagebehälters gegen ein Platzen und Auslaufen zu erhöhen, absolut kontraproduktiv zu sein.

In aufwändigen Versuchsreihen hat sich jedoch überraschend gezeigt, dass durch Spritzblasformen hergestellte Wunddrainagebehälter eine äußerst homogene Wandstärke aufweisen, wodurch lokale Schwachstellen, wie sie beim Extrusionsblasformen festgestellt und als Ausgangspunkt eines Versagens des Wunddrainagebehälters identifiziert werden konnten, verhindert werden. In anderen Worten ausgedrückt hat sich herausgestellt, dass die verfahrensspezifische Homogenisierung der Wandstärke die verfahrensspezifische Wandstärkenreduktion hinsichtlich der Auslaufsicherheit deutlich mehr als kompensiert und so ein insgesamt sicheres Behältnis erzeugt wird.

Ein weiterer Vorteil des durch Spritzblasformen hergestellten Wunddrainagebehälters ist die geringere Gratbildung, insbesondere im Vergleich zu einer Herstellung mittels Extrusionsblasformen. Der Grat kann als Keimstelle des Versagens des Wunddrainagebehälters fungieren. Daher ist ein geringer Grat vorteilhaft für die Sicherheit des Wunddrainagebehälters gegen Auslaufen oder Platzen.

Kern der Erfindung ist deshalb, dass ein mittels Spritzblasformen hergestellter Wunddrainagebehälter trotz der geringeren Wandstärke überraschenderweise eine höhere Sicherheit gegen Platzen/Versagen bietet als ein konventioneller Wunddrainagebehälter mit dickerer Wandstärke, der mittels Extrusionsblasformen hergestellt ist.

In einem ersten Aspekt kann der Behälterkörper eine homogene Wandstärke in einem Bereich von 1,9 mm bis 2,2 mm aufweisen.

In anderen Worten ist die Wandstärke des Behälterkörpers an jeder Stelle größer gleich 1,9 mm und kleiner gleich 2,2 mm. Durch die homogene Wandstärke ist das Risiko einer Rissbildung in einer Wand des Wunddrainagebehälters an einer lokalen Schwachstelle mit reduzierter Wandstärke erheblich reduziert. So schwankt die Wandstärke bei mittels Extrusionsblasformen hergestellten Wunddrainagebehältern beispielsweise in einem Bereich von 1,3 mm bis 3,5 mm, was erhebliche Nachteile in der Festigkeit mit sich bringt, und an Stellen mit großer Wandstärkeänderung wie beispielsweise an den Übergängen zu einem Boden des Wunddrainagebehälters für Spannungsspitzen sorgt, die ein Versagen des Wunddrainagebehälters weiter begünstigen.

Zusätzlich ist durch die geringere Wandstärke das Gewicht des mittels Spritzblasformen hergestellten Wunddrainagebehälters zwischen 10 % und 20 % geringer als das Gewicht eines mittels Extrusionsblasformen hergestellten Wunddrainagebehälters mit gleicher Kapazität, was die Handhabung des Wunddrainagebehälters erleichtert.

Erfindungsgemäß ist der Deckel mittels Spritzgießen hergestellt.

In anderen Worten wird der Deckel mittels einem Urformenden Verfahren aus Kunststoff hergestellt. Hierfür wird der Kunststoff in einer Spritzeinheit plastifiziert und in ein Spritzgießwerkzeug eingespritzt. Das Spritzgießwerkzeug weist hierfür einen Hohlraum (Kavität) auf, die der (Negativ-) Form des zu formenden Deckels entspricht. Beim Spritzgießen handelt es sich um ein etabliertes Verfahren, das gut beherrschbar ist und reproduzierbare konstante Ergebnisse liefert.

In einem weiteren Aspekt kann der Deckel und der Behälterkörper mittels Ultraschallschweißen verbunden sein.

In anderen Worten werden der Deckel und der Behälterkörper durch mechanische Schwingungen, vorzugsweise in einem Bereich zwischen 20 kHz und 35 kHz, welche zwischen dem Deckel und dem Behälterkörper zu Erwärmung durch Molekular- und Grenzflächenreibung führt. Die Fügepartner in Form von dem Deckel und dem Behälterkörper erwärmen sich lokal und schmelzen lokal auf. Die aufgeschmolzenen Werkstoffe verbinden sich und sind nach dem Abkühlen und Erstarren miteinander stoffschlüssig verbunden/verschweißt. Durch ein Verschweißen des Deckels mit dem Behälterkörper kann ein Auslaufen des Wunddrainagebehälters vermieden werden.

In einem weiteren Aspekt können der Deckel und der Behälterkörper mittels eines Gewindes verbunden sein.

In anderen Worten ist an einer Öffnung des Behälterkörpers vorzugsweise ein Außengewinde vorgesehen und an dem Deckel ein entsprechendes Innengewinde. Das Gewinde des Behälterkörpers ist vorzugsweise bereits bei dem Vorformling ausgebildet. So kann die Genauigkeit des Gewindes erhöht werden, was eine Handhabung des Wunddrainagebehälters vereinfacht.

In einem weiteren Aspekt können der Deckel und der Behälterkörper mittels Kleben verbunden sein.

In anderen Worten ist an der Öffnung des Behälterkörpers eine erste Klebefläche ausgebildet und an dem Deckel eine zweite Klebefläche. Durch ein flächiges Verkleben des Deckels mit dem Behälterkörper kann ein Auslaufen des Wunddrainagebehälters vermieden werden.

In einem weiteren Aspekt kann der Behälterkörper aus Polyethylenterephthalat (PET) gebildet sein.

In anderen Worten besteht der Behälterkörper stoffeinstückig aus Polyethylenterephthalat (PET). Polyethylenterephthalat weist eine hohe Festigkeit auf. Zusätzlich besitzt Polyethylenterephthalat eine gute chemische Beständigkeit gegen Säuren und ist bis 70 °C hydrolysebeständig. Ein weiterer Vorteil von Polyethylenterephthalat ist, dass er eine hohe Transparenz aufweist. Aufgrund dieser Eigenschaften eignet sich Polyethylenterephthalat besonders für den Einsatz als Wunddrainagebehälter.

In einem weiteren Aspekt kann der Deckel aus Polyamid (PA) gebildet sein.

In anderen Worten besteht der Deckel stoffeinstückig aus Polyamid (PA). Polyamid weist eine hohe Formbeständigkeit auf, ist robust und lässt sich gut im Spritzgießverfahren verarbeiten. Zusätzlich ist Polyamid mit Polyethylenterephthalat schweißbar.

Weiterhin offenbart die vorliegende Erfindung einen zweiteiligen Wunddrainagebehälter mit einem Behälterkörper und einem Deckel, wobei der Behälterkörper aus einem vorzugsweise spritzgegossenen Vorformling auf einem Spritzdorn in einer Blasform mittels Druckluft in eine Form/Geometrie geblasen wird. Vorzugsweise ist ein Gewinde, das vorzugsweise an einer Öffnung des Behälterkörpers ausgebildet ist, an dem spritzgegossenen Vorformling bereits ausgebildet. Kurzbeschreibung der Figuren
Fig. 1 ist eine perspektivische Darstellung eines erfindungsgemäßen Wunddrainagebehälters.
Fig. 2 ist eine seitliche Darstellung des erfindungsgemäßen Wunddrainagebehälters.
Fig. 3 ist eine Schnittdarstellung entlang eines in Fig. 2 eingezeichneten Schnitts A-A des erfindungsgemäßen Wunddrainagebehälters.
Fig. 4 ist eine in Fig. 3 eingezeichnete Detailansicht B eines Deckels und einer Verschraubung eines erfindungsgemäßen Wunddrainagebehälters.

### Detaillierte Beschreibung anhand der Figuren

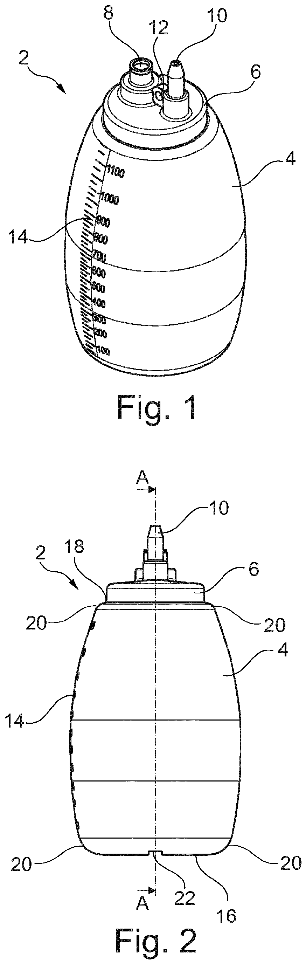

Fig. 1 zeigt einen Behälter, der erfindungsgemäß als Wunddrainagebehälter 2 verwendet wird. Der Wunddrainagebehälter 2 beinhaltet einen Behälterkörper 4 und einem Deckel 6. Der Deckel 6 beinhaltet einen ersten Anschluss 8, einen zweiten Anschluss 10 und eine Befestigungsöse 12. Der erste Anschluss 8 und der zweite Anschluss 10 sind dafür vorgesehen und ausgebildet, mit einem Wunddrainageschlauch (nicht dargestellt), der eine Drainageflüssigkeit (Wundflüssigkeit, Blut und dergleichen) von einer in einer Wunde ausgebildeten Wunddrainage in den Wunddrainagebehälter 2 zu leiten, verbunden zu sein. Alternativ ist nur entweder der erste Anschluss 8 oder der zweite Anschluss 10 mit dem Wunddrainageschlauch verbunden und der jeweils andere Anschluss ist mit einem Stopfen verschlossen. Nochmals alternativ ist an dem ersten Anschluss 8 der Wunddrainageschlauch angeschlossen und an dem zweiten Anschluss 10 eine Vakuumpumpe. In einer bevorzugten Ausführungsform sind die Anschlüsse als Luer Lock Konnektoren ausgeführt. Die Befestigungsöse 12 ist vorgesehen und ausgebildet, mit einem Haken oder eine Schlaufe durchzogen zu werden, sodass der Wunddrainagebehälter 2 beispielsweise an einem Patientenbett aufgehängt oder von dem Patienten mittels der Schlaufe komfortabel getragen werden kann.

Der Behälterkörper 4 weist vorzugsweise eine fassförmige Form auf. Anders ausgedrückt ist die Wand des Behälterkörpers 4 in einer Behälterhöhenrichtung gekrümmt. Vorzugsweise ist die Krümmung über die Höhe des Behälterkörpers 4 konstant. Alternativ ist der Behälterkörper 4 in Höhenrichtung in mehrere Abschnitte unterteilt, die sich in ihrer Krümmung unterscheiden. Durch die Krümmung ist die Stabilität des Behälters erhöht. Weitere bevorzugte Formen des Behälterkörpers 4 sind eine ballonförmige Form, eine zylindrische Form oder eine gewinkelte Form, bei der mehrere gerade Wandelemente über vorzugsweise stumpfe Winkel miteinander verbunden sind.

Zusätzlich ist auf der Wand des Behälterkörpers 4 eine Füllstandskala 14 vorgesehen und ausgebildet. Durch den transparenten, vorzugsweise aus Polyethylenterephthalat gefertigten Behälterkörper 4 ist so die Menge an Wunddrainageflüssigkeit, die sich in dem Wunddrainagebehälter befindet, an der Füllstandskala 14 ablesbar.

Fig. 2 ist eine Seitenansicht des Wunddrainagebehälters 2. Die Übergänge von der Wand des Behälterkörpers 4 zu einem Boden 16 und einer Oberseite 18 des Behälterkörpers 4 sind als Radien 20 ausgebildet. Die Radien 20 reduzieren Spannungen und ermöglichen eine gleichmäßige Kraftleitung in dem Behälterkörper 4 des Wunddrainagebehälters 2. Der Boden 16 ist optional mit einer Verstärkungssicke 22 ausgebildet, die vorgesehen und ausgebildet ist, die Steifigkeit des Bodens 16 zu erhöhen.

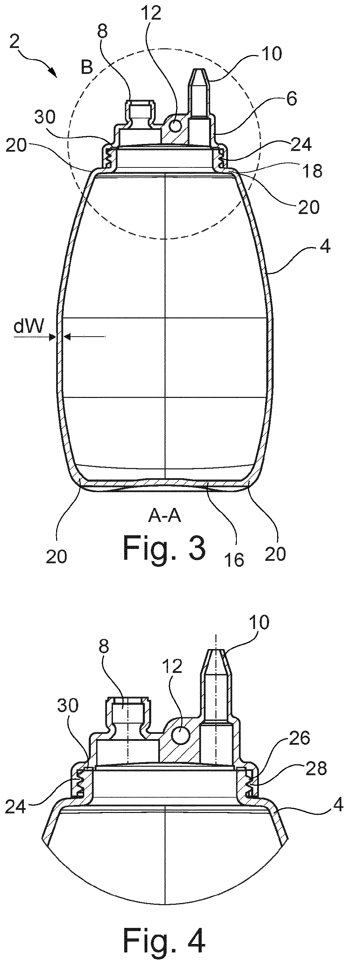

Fig. 3 zeigt den in Fig. 2 dargestellten Schnitt A-A durch den Wunddrainagebehälter 2. Eine Wandstärke dW ist über den gesamten Behälterkörper 4 homogen. Bevorzugt liegt die Wandstärke dW über den gesamten Behälterkörper 4 in einem Bereich von 1,9 mm bis 2,2 mm. Anders ausgedrückt ist die Wandstärke dW in dem Boden 16, der Oberseite 18 und der den Boden 16 und die Oberseite 18 verbindenden Wand in dem Bereich zwischen 1,9 mm bis 2,2 mm. Insbesondere auch in den Radien 20 liegt die Wandstärke in dem Bereich zwischen 1,9 mm bis 2,2 mm.

An der Oberseite 18 des Behälterkörpers 4 ist eine Öffnung ausgebildet. Zwischen Behälterkörper 4 und Deckel ist eine Schraubverbindung 24 vorgesehen und ausgebildet, welche den Behälterkörper 4 mit dem Deckel 6 verbindet. Alternativ kann die Verbindung von Deckel 6 und Behälterkörper 4 als Klebeverbindung ausgeführt sein. Im Falle einer Klebeverbindung ist sowohl an dem Deckel 6 als auch an dem Behälterkörper 4 eine Klebefläche ausgebildet, die in einem verbundenen Zustand in Deckung liegen und mittels eines Klebstoffes stoffschlüssig verbunden sind. Weiterhin alternativ kann die Verbindung von Deckel 6 und Behälterkörper 4 als (Ultraschall-) Schweißverbindung ausgeführt sein. Im Falle einer Schweißverbindung ist sowohl an dem Deckel 6 als auch an dem Behälterkörper 4 eine Schweißfläche ausgebildet, die in einem verbundenen Zustand in Deckung liegen und durch lokales Aufschmelzen stoffschlüssig miteinander verbunden sind.

Fig. 4 ist eine vergrößerte Ansicht des Abschnitts B des Schnitts A-A aus Fig. 3. Der Deckel 6 ist vorzugsweise mittel Spritzgießen hergestellt und besteht vorzugsweise aus Polyamid. Der Deckel weist ein Innengewinde 26 auf, das mit dem Außengewinde 28 des Behälterkörpers 4 im Eingriff steht. Innengewinde 26 und Außengewinde bilden die Schraubverbindung 24. Zwischen Deckel 6 und Behälterkörper ist zusätzlich eine Dichtung 30 ausgebildet. Die Dichtung 30 ist vorzugsweise in einer Nut ausgebildet, die sich in dem Deckel 6 befindet und gegen eine Dichtfläche, die sich an dem Behälterkörper 4 befindet, abdichtet. Das Außengewinde 28 ist vorzugsweise bereits auf dem Vorformling ausgebildet, was die Maßhaltigkeit des Außengewindes 28 erheblich steigert.

Weiteren relevante Materialien, die entweder für die Behälterkörper 4 oder den Deckel 6 eingesetzt werden können sind Styrol-Acrylnitril-Copolymere, Polycarbonat, Methylmethacrylat, Polypropylen oder ein beliebiges Copolymer.

### Bezugszeichenliste

- 2: Wunddrainagebehälter
- 4: Behälterkörper
- 6: Deckel
- 8: erster Anschluss
- 10: zweiter Anschluss
- 12: Befestigungsöse
- 14: Füllstandskala
- 16: Boden
- 18: Oberseite
- 20: Radien
- 22: Verstärkungssicke
- 24: Schraubverbindung
- 26: Innengewinde
- 28: Außengewinde
- 30: Dichtung
- dW: Wandstärke

## Patentansprüche

1. Wunddrainagebehälter (2), welcher vorgesehen und ausgebildet ist, Wundflüssigkeit aufzunehmen, mit einem Behälterkörper (4) und einem Verschlussdeckel (6), **dadurch gekennzeichnet, dass** der Verschlussdeckel (6) mittels Spritzgießen hergestellt ist und einen ersten Anschluss (8) und einen zweiten Anschluss (10) aufweist und der Behälterkörper (4) durch Kunststoff-Spritzblasformen hergestellt ist.

2. Wunddrainagebehälter (2) nach Anspruch 1, wobei der Behälterkörper (4) eine homogene Wandstärke (dW) in einem Bereich von 1,9 mm bis 2,2 mm aufweist.

3. Wunddrainagebehälter (2) nach einem der Ansprüche 1 oder 2, wobei Deckel (6) und Behälterkörper (4) mittels Ultraschallschweißen verbunden sind.

4. Wunddrainagebehälter (2) nach einem der Ansprüche 1 oder 2, wobei der Deckel (6) und der Behälterkörper (4) mittels eines Gewindes verbunden sind.

5. Wunddrainagebehälter (2) nach einem der Ansprüche 1 oder 2, wobei der Deckel (6) und der Behälterkörper (4) mittels Kleben verbunden sind.

6. Wunddrainagebehälter (2) nach einem der Ansprüche 1 bis 5, wobei der Behälterkörper (4) aus Polyethylenterephthalat (PET) gebildet ist.

7. Wunddrainagebehälter (2) nach einem der Ansprüche 1 bis 6, wobei der Deckel (6) aus Polyamid (PA) gebildet ist.

## Claims

1. A wound drainage container (2), which is intended and configured to receive wound fluid, comprising a container body (4) and a closing lid (6), **characterized in that** the closing lid (6) is produced by injection molding and has a first terminal (8) and a second terminal (10), and the container body (4) is produced by plastic injection blow molding.

2. The wound drainage container (2) according to claim 1, wherein the container body (4) has a homogeneous wall thickness (dW) in a range from 1.9 mm to 2.2 mm.

3. The wound drainage container (2) according to one of claims 1 or 2, wherein lid (6) and container body (4) are connected via ultrasonic welding.

4. The wound drainage container (2) according to one of claims 1 or 2, wherein the lid (6) and the container body (4) are connected via a thread.

5. The wound drainage container (2) according to one of claims 1 or 2, wherein the lid (6) and the container body (4) are connected via adhesive bonding.

6. The wound drainage container (2) according to one of claims 1 to 5, wherein the container body (4) is formed from polyethylene terephthalate (PET).

7. The wound drainage container (2) according to one of claims 1 to 6, wherein the lid (6) is formed from polyamide (PA).

## Revendications

1. Récipient de drainage pour plaies (2) qui est prévu et conçu pour recevoir du liquide de plaie, avec un corps de récipient (4) et un couvercle de fermeture (6), **caractérisé en ce que** le couvercle de fermeture (6) est fabriqué au moyen d'un moulage par injection et présente un premier raccordement (8) et un second raccordement (10) et le corps de récipient (4) est fabriqué par moulage par injection-soufflage de plastique.

2. Récipient de drainage pour plaies (2) selon la revendication 1, dans lequel le corps de récipient (4) présente une épaisseur de paroi homogène (dW) située dans une plage allant de 1,9 mm à 2,2 mm.

3. Récipient de drainage pour plaies (2) selon l'une quelconque des revendications 1 ou 2, dans lequel le couvercle (6) et le corps de récipient (4) sont reliés au moyen d'une soudure par ultrasons.

4. Récipient de drainage pour plaies (2) selon l'une quelconque des revendications 1 ou 2, dans lequel le couvercle (6) et le corps de récipient (4) sont reliés au moyen d'un filetage.

5. Récipient de drainage pour plaies (2) selon l'une quelconque des revendications 1 ou 2, dans lequel le couvercle (6) et le corps de récipient (4) sont reliés au moyen d'un collage.

6. Récipient de drainage pour plaies (2) selon l'une quelconque des revendications 1 à 5, dans lequel le corps de récipient (4) est formé à partir de téréphtalate de polyéthylène (PET).

7. Récipient de drainage pour plaies (2) selon l'une quelconque des revendications 1 à 6, dans lequel le couvercle (6) est formé à partir de polyamide (PA).
